# EUROPÄISCHE PATENTSCHRIFT

(11) **EP 1 014 807 B1**
(45) Veröffentlichungstag und Bekanntmachung des Hinweises auf die Patenterteilung: **31.10.2001**
(21) Anmeldenummer: 98949979.3
(22) Anmeldetag: 28.08.1998
(51) Int. Cl.: A23K 3/03, A23L 3/3508, A23B 4/12, A23B 4/023

(54) **GETRÄNKTE SALZE, EIN VERFAHREN ZU IHRER HERSTELLUNG UND IHRE VERWENDUNG**
IMPREGNATED SALTS, A METHOD FOR PRODUCING SAID SALTS, AND THE USE OF THE SAME
SELS IMPREGNES, LEUR PROCEDE DE PRODUCTION ET LEUR UTILISATION

(30) Priorität: 08.09.1997 DE 19739319
(43) Veröffentlichungstag der Anmeldung: 05.07.2000
(73) Patentinhaber: BASF AKTIENGESELLSCHAFT, 67056 Ludwigshafen (DE)
(72) Erfinder: BRÖCKEL, Ulrich, D-67251 Freinsheim (DE); KAESLER, Bruno, D-67071 Ludwigshafen (DE); GAUS, Günter, D-68647 Biblis (DE); MEYER, Joachim, D-67133 Maxdorf (DE)
(86) Internationale Anmeldenummer: EP9805469
(87) Internationale Veröffentlichungsnummer: WO9912432

(56) Entgegenhaltungen:
- EP-A- 0 219 997
- EP-A- 0 590 856
- EP-A- 0 608 975
- WO-A-96/35337
- FR-A- 2 368 228
- US-A- 4 199 606
- US-A- 5 547 987

## Beschreibung

Die vorliegende Erfindung betrifft getränkte Salze, enthaltend mindestens ein Salz einer oder mehrerer organischer Carbonsäuren, in das 0,5 bis 30 Gew.-% mindestens einer organischen Carbonsäure bezogen auf das Carbonsäuresalz eingelagert wurde. Konservierungsstoffe enthaltend ein getränktes Salz und gegebenenfalls mindestens einen Trägerstoff und/oder Formulierungshilfsstoffe, wobei die Konservierungsstoffe mit einem Abdeckmittel und/oder einem Puderungsmittel überzogen sein können.

Die Erfindung betrifft weiterhin ein Verfahren zur Herstellung der getränkten Salze und der Konservierungsstoffe, sowie die Verwendung der Salze und Konservierungsstoffe zur Behandlung von Lebens- und Futtermitteln, sowie zur Anwendung in Silagen.

Kurzkettige organische Säuren, wie Ameisensäure, Essigsäure oder Propionsäure finden in der Ansäuerung und Konservierung von Lebens- und Futtermitteln Anwendung. Nachteile dieser Säuren sind beispielsweise ihr flüssiger Aggregatzustand bei Raumtemperatur, der aus dem niedrigen Dampfdruck resultierende scharfe, stechende Geruch und ihre Korrosivität.

Ferner sind die flüssigen organischen Säuren in konzentrierter Form nur durch erheblichen technischen Aufwand beispielsweise in Futtermittel einzuarbeiten.

Aus der DE 28 33 727 A1 ist ein teilchenförmiges, fungizid wirkendes Material bekannt, das Propionsäure und ein Trägermaterial enthält. Mit diesem Material soll auch bei mehrtägiger Inkubationsdauer die Zahl der Schimmelkolonien bei gelagerten landwirtschaftlichen Ernteprodukten nicht ansteigen. Es hat sich aber gezeigt, daß derartiges Material selbst nicht lagerstabil ist (Säureverlust) und die Höchstmenge an Propionsäure, die aufgebracht werden kann, stark vom verwendeten Trägermaterial abhängt. Zudem treten mit diesem Material durch Verflüchtigung der Propionsäure unangenehme Gerüche auf.

WO 96/35 337 beschreibt u.a. Doppelsalze bestchend aus Kalium-, Natnium-, oder Ammoniumformiat und Ameisensäure in äquimolaren Verhältnis. Die Salze werden als Futtermittelzusätze verwendet.

Aus EP-A-0 590 856 und EP-A-0 608 975 sind Mischungen aus festen Carbonsäuresalzen und festen Carbonsäuren mit einem geringeren pKs-Wert als die Carbonsäure in den vorgelegten, verwendeten Salzen bekannt. Durch Lösen dieser Mischung in Wasser werden die Carbonsäuren aus den Salzen in einer Verdrängungsreaktion durch die Carbonsäure mit dem niedrigeren pKs-Wert freigesetzt. Vorteilhafterweise sind die entstehenden neuen Salze mit der Carbonsäure mit dem niedrigeren pKs-Wert wasserunlöslich und fallen in der Lösung aus. Von Nachteil bei diesen Mischungen ist, daß immer verschiedene Carbonsäuren mit unterschiedlichen pKs-Werten für die Herstellung der Konservierungsmittel verwendet werden müssen. Um sicherzustellen, daß aus den vorgelegten Carbonsäuresalzen die Carbonsäuren (beispielsweise aus CaPropionat) bei Lösung in Wasser vollständig freigesetzt werden, müssen die Carbonsäuren mit den niedrigeren pKs-Wert (beispielsweise Maleinsäure) mindestens in äquimolaren Mengen bezogen auf die in den Säuren vorhandenen Carboxylgruppen zugesetzt werden. Dadurch ist der Wirkstoffgehalt der einzelnen Carbonsäure limitiert. Werden bei dieser Freisetzung unlösliche Carbonsäuresalze gebildet, so müssen diese darüberhinaus in einer anschließenden Reaktion abgetrennt werden.

Der vorliegenden Erfindung lag die Aufgabe zugrunde, neue Mittel zur Behandlung von Lebens- und Futtermitteln zur Verfügung zu stellen, die die oben genannten Nachteile nicht aufweisen und vom Anwender leicht und ohne Probleme unter die zu behandelnden Lebens- und Futtermittel gemischt werden können. Dabei stand die Herstellung in Form eines "festen Mittels" mit einem möglichst hohen Wirkstoffgehalt, das keine oder nur eine sehr geringe Geruchsentwicklung zeigt, im Vordergrund. Das feste Endprodukt sollte gute Lager-, Fließ- und Verarbeitungseigenschaften aufweisen.

Diese Aufgabe wurde durch die erfindungsgemäßen getränkten Salze, enthaltend mindestens ein Salz einer oder mehrerer organischer Carbonsäuren, das mit 0,5 bis 30 Gew.-% mindestens einer flüssigen Carbonsäure bezogen auf das Carbonsäuresalz getränkt wurde, gelöst.

Die Erfindung betrifft außerdem Konservierungsstoffe, enthaltend ein getränktes Salz der oben genannten Zusammensetzung. Zusätzlich können die Konservierungsstoffe vorteilhafterweise mindestens einen Trägerstoff und/oder Formulierungshilfsstoff enthalten und gegebenenfalls mit einem Abdeckmittel und/oder Puderungsmittel überzogen sein.

Die Erfindung betrifft weiterhin ein Verfahren zur Herstellung der getränkten Salze, dadurch gekennzeichnet, daß man mindestens ein Salz einer Carbonsäure oder eines Carbonsäuregemisches mit mindestens einer flüssigen Carbonsäure bis zu einer Konzentration von 30 Gew.-% bezogen auf das Carbonsäuresalz getränkt wurde.

Außerdem betrifft die Erfindung die Herstellung von Konservierungsstoffen, enthaltend ein getränktes Salz der oben genannten Zusammensetzung, dadurch gekennzeichnet, daß man getränkte Salze, enthaltend mindestens ein Salz einer oder mehrerer organischer Carbonsäuren, das mit 0,5 bis 30 Gew.-% mindestens einer flüssigen Carbonsäure getränkt wurde, gegebenenfalls mit mindestens einem Trägerstoff und/oder mindestens einem Formulierungshilfsstoff vermischt und ohne oder unter Zugabe eines oder mehreren Bindemitteln agglomeriert und anschließend die Konservierungsstoffe vorteilhafterweise mit einem Abdeckmittel, das bei Raumtemperatur (23°C) erstarrt, versieht, wobei das Abdeckmittel in solchem Maße zugegeben wird, daß die entstehenden Konservierungsstoffe gecoatet werden und gegebenenfalls eine weitere Odorierung durch beispielsweise die Zugabe von Riechstoffen erfolgt. Die so hergestellten Konservierungsstoffe können zur Verbesserung der Rieselfähigkeit der Konservierungsstoffe zusätzlich noch vorteilhaft mit einem feindispersen Pudermittel überzogen werden.

Die erfindungsgemäßen getränkten Salze oder Konservierungsstoffe haben den Vorteil, daß eine Reduzierung des stark stechenden Säuregeruchs erreicht wird. Die getränkten Salze enthalten vorteilhafterweise einen Wirkstoffgehalt von 68 bis 75 Gew.-%, bevorzugt 70 bis 73 Gew.-%, besonders bevorzugt 70 bis 72 Gew.-%, als Summe bezogen auf die Gesamtcarbonsäuremenge der im Salz enthaltenen und der zugesetzten Carbonsäure. Sowohl die erfindungsgemäßen getränkten Salze als auch die Konservierungsstoffe geben den Säureanteil gut und schnell aus dem Feststoff ab und besitzen eine gute Lager-, Fließ- und Verarbeitungseigenschaft.

Unter "flüssigen organischen Säuren", die zur Tränkung der Carbonsäuresalze geeignet sind, werden Säuren oder Säuregemische verstanden, die bei den Verarbeitungstemperaturen, vorzugsweise bis zu einer Temperatur von 40°C oder darunter, flüssig sind oder flüssig werden.

Vorteilhafterweise werden organische C₁-C₈-Mono- und/oder Di-Carbonsäuren und die Salze dieser C₁-C₈-Mono- und/oder Di-Carbonsäuren zur Herstellung der getränkten Salze oder zur Herstellung der Konservierungsstoffe verwendet. Als Salze sind die Alkali-, Erdalkali- oder Ammoniumsalze geeignet. Bevorzugt werden Säuren wie Ameisen-, Essig- und/oder Propionsäure bzw. deren Ammonium-, Calcium-, Lithium-, Natrium-, Magnesium- und/oder Kaliumsalze verwendet. Vorteilhaft werden die Calcium-, Natrium- oder Ammoniumsalze verwendet. Prinzipiell sind aber auch andere Säuren wie beispielsweise Aminosäuren, Hydroxycarbonsäuren, Oxosäuren oder Mineralsäuren wie HCL oder H₂SO₄ und deren Salze geeignet, wobei Mineralsäuren weniger bevorzugt sind. Es können einzelne Salze oder die Gemische unterschiedlicher Salze einer Carbonsäure oder mehrerer Carbonsäuren, die mit einer oder mehreren Säuren zur Herstellung der getränkten Salze getränkt wurden, verwendet werden. Vorteilhafterweise bestehen die getränkten Salze aus den Salzen einer Carbonsäure, die mit der gleichen Carbonsäure getränkt wurde. Bevorzugt bestehen die getränkten Salze aus dem Salz einer Carbonsäure, das mit der gleichen Carbonsäure getränkt wurde. Besonders bevorzugt sind Salze der Ameisensäure und/oder Propionsäure, die mit Ameisensäure und/oder Propionsäure in möglichst konzentrierter Form getränkt wurden, beispielsweise mit 99%iger Ameisensäure. Ganz besonders bevorzugt werden getränkte Salze, die aus den Salzen der Ameisensäure und Ameisensäure hergestellt wurden.

Die erfindungsgemäßen getränkten Salze zeigen in der Röntgenstrukturanalyse gegenüber normalen Carbonsäuresalzen eine weitere Bande.

Die getränkten Salze können zur Verbesserung der Handhabbarkeit vorteilhafterweise mit weiteren Stoffen beispielsweise mit einem Träger abgemischt werden und/oder mit einem Puderungsmittel abgepudert werden.

Unter dem Begriff "Tränken" ist zu verstehen, daß mindestens eine bei 40 °C oder oder unter dieser Temperatur flüssige Carbonsäure auf das vorgelegte oder die vorgelegten festen Carbonsäuresalze aufgebracht wird, so daß die flüssige Carbonsäure bzw. Carbonsäuren in die oder in den Salzkristall eindringt. Dies geschieht in der Regel unter leichter Erwärmung. Für das Tränken wird mindestens eine organische Carbonsäure mit bis zu 30 Gew.-% (= 30 Gewichtsanteilen) bezogen auf das Carbonsäure- bzw. die Carbonsäuresalze auf das Salz oder die Salze aufgebracht, bevorzugt wird die Carbonsäure bzw. die Carbonsäuren in einer Menge von 0,5 bis 30 Gew.-%, besonders bevorzugt von 15 bis 25 Gew.-%, ganze besonders bevorzugt von 15 bis 20 Gew.-% bezogen auf die Salzkomponente aufgebracht, so daß als Reaktionsprodukt eine Festsubstanz entsteht. Über 30 Gew.-% Säureanteil beginnen die Salzkristalle zu verkleben; zum Teil liegt neben den getränkten Salzen unter diesen Bedingungen freie Carbonsäure vor. Durch Zumischen eines Trennmittel können diese verklebten Kristalle voneinander getrennt werden und die freie Carbonsäure durch das Trennmittel aufgenommen werden. Über 35 Gew.-% Säureanteil verkleben die Reaktionsprodukte so stark, das sich eine pastenartige Struktur infolge der freien Carbonsäure ergibt. Diese pastenartige Strukturen können beispielsweise in einem weiteren Arbeitsschritt durch Zugabe eines Trennmittels und Bearbeitung in beispielsweise einem Mischer zu einem Granulat verarbeitet werden. Da bei der Zugabe über 30 Gew.-% freier Säure weitere Arbeitsschritte sowie größere Mengen eines Trennmittels erforderlich sind, sind diese Ausführungsformen aus wirtschaftlichen Gründen weniger bevorzugt. Prinzipiell können jedoch zur Verbesserung der Fließfähigkeit der getränkten Salze auch unterhalb von 30 Gew.-% Carbonsäure geringe Mengen eines Trennmittels zugegeben werden. Geeignete vorteilhafte Trennmittel sind beispielsweise Sipernate, Aerosile und/ oder Tixosile.

Im erfindungsgemäßen Verfahren zur Herstellung der getränkten Salze wird also mindestens ein Salz einer Carbonsäure oder eines Carbonsäuregemisches mit mindestens einer bei 40°C oder unter dieser Temperatur flüssigen Carbonsäure bis zu einer Konzentration von 30 Gew.-% bezogen auf das vorgelegte Carbonsäuresalz bzw. die vorgelegten Carbonsäuresalze getränkt.

Im erfindungsgemäßen Verfahren zur Herstellung der getränkten Salze kann auch mindestens eine Carbonsäure vorgelegt werden und mindestens ein Salz einer oder mehrerer organischen Carbonsäuren zugegeben werden. Diese Art der Herstellung ist gegenüber der Vorlage des oder der Salze ungünstiger, so daß unter diesen Bedingungen beispielsweise bei der Herstellung in einem Mischer ein erhöhter Energieeintrag notwendig ist.

Vorteilhafterweise wird das erfindungsgemäße Verfahren zur Herstellung von getränkten Salzen bei einer Temperatur durchgeführt, die durch den Erstarrungspunkt der verwendeten Carbonsäure festgelegt wird. Das Verfahren wird in einem Temperaturbereich von 0 bis 60 °C, bevorzugt von 15 bis 50 °C, besonders bevorzugt von 20 bis 40 °C durchgeführt.

In einer bevorzugten Ausführungsform weist das erfindungsgemäße Salz noch ein Abdeckungsmittel und/oder Puderungsmittel auf der Kristalloberfläche auf. Die Kristallgröße der getränkten Salze liegt vorzugsweise in einem Bereich unterhalb 2,5 mm, bevorzugt zwischen 10 µm und 2000 µm, ganz besonders bevorzugt zwischen 300 µm und 1500 µm.

Unter den erfindungsgemäßen Konservierungsstoffen sind Konservierungsstoffe, enthaltend getränkte Salze, enthaltend mindestens ein Salz einer oder mehrerer organischer Carbonsäure, die mit mindestens einer flüssigen Carbonsäure getränkt wurden, zu verstehen. Diese getränkten Salze können in den Konservierungsstoffen mit einem oder mehreren Trägerstoffen und/oder Formulierungshilfsstoffen vermischt sein. Im erfindungsgemäßen Verfahren zur Herstellung der Konservierungsstoffe kann diese Mischung ohne oder unter Zugabe von Bindemittel agglomeriert werden. Auf diese Konservierungsstoffe kann anschließen noch ein bei 20°C wasserlösliches oder wasserquellbares Abdeckmittel und/oder ein feindisperses Pudermittel aufgetragen werden, so daß die erfindungsgemäßen Konservierungsstoffe eine Hülle aus einem Abdeckmittel und/oder Pudermittel besitzen.

Als Träger können poröse, organische oder anorganische Trägermaterialien eingesetzt werden, deren Partikelgrößen zwischen 1 µm und 1.000 µm, vorzugsweise zwischen 5 µm und 100 µm liegen.

Für die Herstellung von derart rieselfähigen geruchsreduzierten Agglomeraten sind prinzipiell alle bekannten organischen bzw. anorganischen porös Träger geeignet, sofern sie säurebeständig sind. Beispiele sind Getreidekleien, Perlit, Tonmaterialien, Silicate und Kieselsäuren, wobei den anorganischen Trägern der Vorzug zu geben ist, da deren Stoffeigenschaften besser kontrolliert werden können.

Verwendbare weitere Träger sind beispielsweise Diatomeenerde, zerstoßener Sand, Ton, Nylonpulver, unlösliche Metalloxide oder unlösliche Metallsalze, Aerosil, Korund, gemahlenes Glas, Granit, Quarz oder Flint, Aluminiumphosphat, Kaolin, Bentonit, Zeolite, Calciumsilicat, Talkum, Titanoxid, Aktivkohle oder Knochenmehl.

Als Träger werden bevorzugt Getreidekleien, Silikate, Perlit oder Kieselsäuren in Anteilen zwischen 10 Gew.-% und 70 Gew.-%, vorzugsweise mit 20 bis 40 Gew.-%, bezogen auf das Gewicht des getränkten Salzes, verwendet.

Als Bindemittel im erfindungsgemäßen Verfahren kommt Wasser und/oder synthetische oder natürliche Polymere beispielsweise Albumin, Casein, Sojaprotein, Stärke, synthetische Cellulosederivate wie Carboxymethylcellulose, Methylcellulose, Hydroxymethyl-, Hydroxyethyl- und/oder -propylcellulose, Polyethylenglykol, Polyvinylalkohol, Polyvinylpyrrolidon, Gelatine, Carrageen, Chitosan, Dextrin, Alginate, Agar-Agar, Gummi Arabicum, Traganth, Polyvinylalkohole oder Guar Gum oder deren Mischungen in Frage.

Als Abdeckmittel können wasserlösliche Polymere wie synthetische oder natürliche Polymere beispielsweise Gelatine, Carrageen, Alginate oder Polyvinylpyrrolidon, organische Säuren, deren Salze oder niedrig schmelzende anorganische Salze verwendet werden.

Als Abdeckmittel werden bevorzugt Polyethylenglykole, Polyvinylpyrrolidone oder organische Säuren und deren Salze von C₃ bis C₁₄, vorzugsweise C₃ bis C₆, insbesondere Zitronensäure, Fumarsäure, Bernsteinsäure, Adipinsäure, Benzoesäure und deren Salze oder Aminosäuren und deren Salze, verwendet.

In einer bevorzugten Ausführungsform des erfindungsgemäßen Verfahrens zur Herstellung der Konservierungsstoffe wird das Carbonsäuresalz in einem Mischer vorgelegt, mit der organischen Säure getränkt gegebenenfalls ein Träger zugemischt und anschließend mit dem Abdeckmittel in Gegenwart oder Abwesenheit eines Bindemittels agglomeriert und gecoatet.

Die getränkten Salze und/oder Trägerstoffpartikel werden mit dem Abdeckmittel gemischt, wobei das Abdeckmittel in der Regel aus einer hochkonzentrierten Lösung oder Schmelze von wasserlöslichen oder wasserquellbaren Substanzen besteht, die bei Raumtemperatur (23 °C) erstarren. Dieses Abdeckmittel wird bevorzugt im erwärmten Zustand auf die getränkten Salz- und/oder Trägerstoffpartikel aufgebracht und mit diesen gemischt. Dabei erstarrt das Abdeckmittel auf der Oberfläche der getränkten Salze und/oder Trägerpartikel. Durch geeignete Betriebsparameter des Mischers kommt es zur Agglomeration verschiedener Partikeln zu größeren Granulaten.

Die Größe der Granulate kann durch Verfahrensparameter beispielsweise beim Mischen oder bei der Wirbelbettgranulation, wie auch durch die Bindemittelmenge und -art oder auch durch nachherige Siebung oder Mahlung eingestellt werden. Die Granulate haben bevorzugt einen mittleren Durchmesser kleiner 3 mm, insbesondere von 0,3-1,3 mm. Gegebenenfalls kann im Abdeckmittel, das zum Coaten und Agglomerieren dient, restliches Wasser vorhanden sein. Nach dem Agglomerationsvorgang oder direkt nach Herstellung der getränkten Salze kann restliches Wasser durch einen Abpuderungsvorgang mit einem trockenen und feindispersen Puderungsmittel gebunden werden. Durch diesen Abpuderungsvorgang läßt sich auch ein späteres Verkleben der Agglomerate oder getränkten Salze verhindern und zusätzlich z.B. das Salz der verwendeten organischen Säure (z.B. Natrium- oder Calciumformiat oder -propionat) auf das Agglomerat oder die Salze aufbringen. Ferner kann beim Abpuderungsschritt gegebenenfalls ein Riech- oder Geschmacksstoff beigesetzt werden, wie z.B. Vanillin, Tecuaroma, Citral oder Fructin, wodurch eine zusätzliche geruchsüberdeckende und z.B. die Tierfutteraufnahme attraktivierende Wirkung erzielbar wird.

Bevorzugt eingesetzte Abdeckmittel wie Bindeflüssigkeiten sind wasserlösliche oder wasserquellbare Substanzen, die bei Raumtemperatur erstarren. Dadurch kann auf einen nachfolgenden Trocknungsschritt verzichtet werden, bei dem neben einem Lösungsmittel oder dem zusätzlichen Wasser auch z.T. die organische Säure ausdampfen würde.

Besonders geeignete Abdeckmittel für den Agglomerationsprozeß und das Coaten sind solche, die eine Erweichungstemperatur von über 30°C, vorzugsweise über 60°C aufweisen, um eine Verformung der Agglomerate bei höherer Lagertemperatur zu verhindern. Bevorzugt sind solche Abdeckmittel anzuwenden, die darüberhinaus der pH-Wert absenkenden Wirkung der adsorbierten organischen Säure nicht entgegenwirken oder diese gegebenenfalls noch unterstützen oder verstärken.

Als Abdeckmittel eignen sich beispielsweise hochkonzentrierte, erhitzte Zuckerlösungen oder Alkali/Erdalkali/-Formiat/-Acetat/-Propionatlösungen. Durch den abschließenden Bepuderungsschritt kann deren Restwassergehalt aufgenommen werden. Niedrig schmelzende Polyethylenglykole wie z.B. PEG 4000, Schmelzen der Zitronensäure, der Adipinsäure, Fumarsäure oder Benzoesäure bzw. deren Salze, hochkonzentrierten Lösungen von Aminosäuren oder Mischungen dieser Säuren eignen sich bevorzugt als Bindeflüssigkeiten. Man verwendet 0,5 bis 80 % Bindeflüssigkeit, vorzugsweise 10 bis 25 Gew.-%, besonders bevorzugt jedoch 5 bis 15 Gew.-%, bezogen auf das Gewicht des Granulats.

Geeignete Puderungsmittel sind neben den porösen Trägermaterialien selbst feindisperse, gemahlene organische Säuren oder deren Salze, z.B. Na-Formiat, sowie anorganische Salze, Sipernate, Tixosile oder Aerosile. Von den Bepuderungsmitteln werden < 10 %, vorzugsweise zwischen 0,1 und 5 Gew.-% zugegeben.

Im allgemeinen wird mindestens ein Salz einer oder mehrerer organischer Säuren in einem Mischer z.B. einem Eirich-Mischer vorgelegt und bei geringen Energieeinträgen mit mindestens einer organischen Säure getränkt. Man kann jedoch auch so verfahren, daß die Flüssigkeit im Mischer vorgelegt wird und die Salze der Carbonsäuren zudosiert werden. In diesem Fall muß mit höheren Energieeinträgen gearbeitet werden.

Darauf zu achten ist, eine gleichmäßige Tränkung sicherzustellen und lokale Überfeuchtungen, die zur Klumpenbildung führen, zu vermeiden. Nach erfolgter Tränkung liegt im Mischer ein rieselfähiges Carbonsäuresalz in Form von festen Kristallen vor. Die Viskosität der anschließend gegebenenfalls zudosierten Bindemittelflüssigkeit sollte durch eine entsprechende Temperaturwahl so eingestellt werden, daß sie unterhalb von 1.000 mPas, bevorzugt im Bereich < 100 mPas liegt, um bei der Verdüsung eine feine Tropfengrößenverteilung zu erreichen. Bedingt durch den Temperaturunterschied zwischen erwärmter Bindeflüssigkeit und kühleren getränktem Salz erstarren bei dieser bevorzugten Ausführungsform die Bindeflüssigkeitstropfen anfänglich schnell. Im weiteren Verlauf des Agglomerationsprozesses steigt die Temperatur der Schüttung durch den mechanischen und den thermischen Energieeintrag je nach Art der Bindeflüssigkeit um 10 bis 30°C an. Auf den bereits gebildeten Agglomeraten lagern sich weitere Bindeflüssigkeitstropfen an, die zum Teil miteinander koalisieren. Der Energieeintrag steigt während der Agglomeration an.

Abschließend kann mit dem Bepuderungsmittel, wie oben dargestellt, zusätzlich ein Odorierungsmittel zugegeben werden. Hierfür eignen sich prinzipiell eine Vielzahl von Riech- und Geschmacksstoffen, die je nach späterer Verwendung des Agglomerats ausgewählt werden können. Der Anteil dieser Riechstoffe kann < 1 Gew.-%, bevorzugt von 0,05 bis 0,5 Gew.-%, bezogen auf das Granulat, betragen. Die so erzeugten Agglomerate sind staubarm, geruchsreduziert und ihr organischer Säureanteil ist leicht wasserlöslich.

Die erfindungsgemäßen getränkten Salze und/oder Konservierungsstoffe eignen sich zur Säurebehandlung oder zur Konservierung von Lebens- und Futtermitteln, zur Anwendung in Silagen oder zur Lederbehandlung. Unter Lebens- und Futtermittel sind insbesondere zu verstehen Gras, landwirtschaftliche Nutzpflanzen und/oder gemischte Tiernahrung und die zu ihrer Herstellung verwendeten Materialien wie Heu, Gerste, Weizen, Hafer, Roggen, Mais, Reis, Sojabohnen, Zuckerrohrrückstände, Zuckerrohr, Rapssamen, Erdnüsse, Sonnenblumensamen, Buchweizenspreu, Silage, Feuchtgetreide, Hülsen- oder Körnerfrüchte, aber auch Milchaustausch-, Flüssig-, Misch- und Mineralfutter, Fischsilagen oder Fisch-, Fleisch- oder Knochemmehl.

Die erfindungsgemäßen Konservierungsstoffe können noch andere i Additive enthalten, wie z.B. Mineralien, Vitamine, Antibiotika oder Protein-Zusatzstoffe. Insbesondere können in den Konservierungsstoffen weitere Zusätze mit fungizider oder bakterizider Eigenschaft wie Formalin, Ameisensäure, Essigsäure, Propionsäure, Benzoesäure, Sorbinsäure oder Bisulfite enthalten sein.

Die erfindungsgemäßen getränkten Salze und/oder Konservierungsstoffe werden dem zu konservierenden Gut vorteilhafterweise in einer Menge von jeweils 0,1 kg bis 25 kg, bevorzugt von 0,5 kg bis 20 kg, besonders bevorzugt von 5 bis 15 kg pro Tonne Konservierungsgut zu gesetzt.

### Beispiele

### (Gehalt der eingesetzten Ameisensäure = 99 %/Propionsäure = 99 %)

### A. Ameisensäure

### Beispiel 1

In einem Haushaltsmischer (Fa. Braun) wurden 100 g Na-Formiat vorgelegt und mit 15 Gew.-% Ameisensäure versetzt. Die Säure wurde unter leichtem Temperaturanstieg von 22°C auf 40°C aufgenommen. Das entstandene Produkt (= getränktes Salz) war rieselfähig und geruchsfrei.

### Beispiel 2

In einem Haushaltsmischer wurden 100 g Ca-Formiat vorgelegt und mit 15 Gew.-% Ameisensäure versetzt. Das entstandene Produkt hatte einen leicht stechenden Geruch nach Ameisensäure und zeigte ein kohäsives Verhalten, das heißt das entstandene getränkte Salz war leicht feucht und nicht gut rieselfähig.

### Beispiel 3

In einem Haushaltsmischer wurden 100 g K-Formiat vorgelegt und mit 10 Gew.-% Ameisensäure versetzt. Die Säure wurde unter leichtem Temperaturanstieg von 23°C auf 45°C aufgenommen. Das getränkte Salz hatte einen leicht stechenden Geruch und zeigte eine leichte Neigung zur Granulation.

### B. Propionsäure

### Beispiel 4

In einem Haushaltsmischer wurden 100 g Fumarsäure vorgelegt und mit 15 Gew.-% Propionsäure versetzt. Das Produkt war stark kohäsiv und hatte einen intensiven Geruch.

### Beispiel 5

Analog den vorherigen Beispielen wurden in einem Haushaltsmischer 100 g Na-Formiat vorgelegt und mit 15 Gew.-% Propionsäure versetzt. Das Produkt ist stark kohäsiv und hat einen intensiven Geruch.

### Beispiel 6

Zu 100 g in einem Haushaltsmischer vorgelegten Ca-Formiat wurden 15 Gew.-% Propionsäure gegeben. Die Säure wurde unter leichtem Temperaturanstieg aufgenommen. Das Produkt hatte einen intensiven Geruch und zeigte ein kohäsives Verhalten.

### Beispiel 7

In einem Haushaltsmischer wurden zu 100 g vorgelegtem CaPropionat 15 Gew.-% Propionsäure gegeben. Die Säure wurde wieder unter Temperaturanstieg von 23°C auf 29°C aufgenommen. Das Produkt ist rieselfähig und hat einen intensiven Geruch.

### C. Herstellung der Konservierungsstoffe

### Beispiel 8

In einem Eirich-Mischer (RO2) wurden 1000 g Na-Formiat vorgelegt und mit 15 Gew.-% Ameisensäure getränkt. Zu 1.000 g dieser Mischung werden als Bindeflüssigkeit 200 g Na-Formiat-Schmelze bei 80°C aus einem beheizten Vorlagebehälter über eine Zweistoffdüse in den Mischraum eingedüst. Die entstandenen Agglomerate werden mit 44 g Sipernat® (= hochdisperse Kieselsäure, Fa. Degussa) abgepudert. Das entstandene Produkt ist rieselfähig und geruchsfrei.

### Beispiel 9

In einem Eirich-Mischer wurden 1000 g Na-Formiat vorgelegt und mit 15 Gew.-% Ameisensäure getränkt. Zum Agglomerieren und Coaten werden als Bindeflüssigkeit 180 g einer konzentrierten Traubenzuckerlösung bei 80°C aus einem beheizten Vorlagebehälter über eine Zweistoffdüse in den Mischraum eingedüst. Die entstandenen Agglomerate werden mit 45 g Sipernat® und 12 g Citral abgepudert. Der Säuregehalt liegt dann bei 59,5 %. Die entstandenen Agglomerate sind gut rieselfähig.

### Beispiel 10

Analog zu Beispiel 9 wurden im Eirich-Mischer 500 g Na-Formiat vorgelegt und mit 15 Gew.-% Ameisensäure getränkt. Anschließend wurden 500 g Perlit zugegeben. Als Bindeflüssigkeit werden 260 g Zitronensäureschmelze bei 170°C aus einem beheizten Vorlagebehälter über eine Zweistoffdüse in den Mischraum eingedüst. Die entstandenen Agglomerate werden mit 44 g Sipernat® und 8 g Vanillin abgepudert. Der Gesamtsäuregehalt liegt bei 29,7 %. Die entstandenen Konservierungsstoffe sind gut rieselfähig und geruchsfrei. Analog zu den Beispielen 8 bis 10 ließen sich auch die in den Beispielen 1 bis 7 beschriebenen getränkten Salze in rieselfähige, geruchsreduzierte oder geruchsfreie Konservierungsstoffe überführen.

Die folgenden Beispiele 11 bis 14 zeigen beispielhaft Lagerstabilitätstests für ein getränktes Salz (NaFormiat mit 20 Gew.-% Ameisensäure getränkt), das mit verschiedenen Abdeckmittel und/oder Pudermittel behandelt wurde (siehe Tabelle 1). Teilmengen der getränkten Salze wurde in einem Taumelmischer gegeben und 10 min unter Zugabe der Additive (siehe Tabelle 1) weiter gemischt. Anschließend wurde ein Stahlgefäß (Durchmesser ca. 40 mm) mit den Produkten bis 15 - 20 mm unterhalb des Gefäßrandes gefüllt und in einem Trockenschrank bei 35°C unter Last (simuliert mit einem Metallstempel) gelagert, wobei die Last einer simulierten Lagerung unter üblichen Lagerungsbedingungen entspricht, und zu den angegebenen Zeiten getestet. Aufgrund des geringen Durchmessers des Testgefäßes und der Lagerung unter Druck ist es für die Ermittlung ob ein Produkt frei fließend ist oder nicht erforderlich, daß man an das Gefäß klopft. Die im Ausflußtest verwendeten Bezeichnungen, die die ermittelten Ergebnis der Versuche wiedergeben, haben die folgende Bedeutung:
1x leicht klopfen, 2x leicht klopfen und 1x klopfen = Produkt frei fließend
3x klopfen = Produkt zeigt Verbackungen, ist jedoch im wesentlichen frei fließend
4x klopfen, 5x klopfen und >5x klopfen = Produkt ist verbacken und im wesentlichen nicht mehr frei fließen

**Tabelle 1:**

| Lagerstabilität der getränkten Salze | | | | |
|---|---|---|---|---|
| Beispiel | Lagerzeit in Tagen | Additiv | Ausflußtest | Bemerkungen |
| 11 | 7 d | 1% FK500LS¹ | 1x klopfen | leicht verbacken, locker |
| | | 2% FK500LS | 1x klopfen | keine Verbackung |
| | | 4% FK500LS | 1x leicht klopfen | keine Verbackung |
| | | 1% Sip.50S² | 2x leicht klopfen | verbacken |
| | | 2% Sip.50S | 1x leicht klopfen | keine Verbackung, staubt |
| | | 4% Sip.50S | 1x leicht klopfen | keine Verbackung, staubt |
| 12 | 14 d | 1% Aerosil200³ | 3x klopfen | verbacken |
| | | 2% Aerosil200 | 2x klopfen | leicht verbacken |
| | | 3% Aerosil200 | 1x klopfen | keine Verbackung |
| | | 4% Aerosil200 | - | |
| | | 5% Aerosil200 | 2x klopfen | keine Verbackung, staubend |
| | | 2% Aerosil200 + 2% D17⁴ | 2x klopfen | keine Verbackung, stark staubend |
| | | 2% D17 | 1x klopfen | leichte Verbackung, staubend |
| | | 5% D17 | 1x klopfen | keine Verbackungen, staubend |
| 13 | 7 d | 2% R972⁵ + 2% Benzoesäure | >5x klopfen | stark verbacken |
| | | 2% R972 + 2% Na-Benzoesäure | >5x klopfen | stark verbacken |
| | | 2% R972 + 2% K-Sorbat | >5x klopfen | stark verbacken |
| | | 4% R972 | 4x klopfen | verbacken |
| | | 2% R972 + 1% FK500LS | 1x klopfen | keine Knollen |
| | | 2% R972 + 2% FK500LS | 1x klopfen | staubt, keine Knollen |
| | | 2% R972 + 2% Zeolith⁶ | 5x klopfen | stark verbacken |
| | | 2% R972 + 2% Sorbit | >5x klopfen | stark verbacken |
| 14 | 7 | 1 Gew.-% Aerosil200 | 3x klopfen | verbacken |
| | | 2 Gew -% Aerosil200 | 2x klopfen | leicht verbacken |
| | | 3 Gew.-% Aerosil200 | 1x klopfen | leicht verbacken |
| | | 4 Gew.-% Aerosil200 | 3x klopfen | nicht verbacken, |
| | | 5 Gew.-% Aerosil200 | 3x klopfen | nicht verbacken, staubend |
| | | 2 Gew.-% Maismehl | >5x klopfen | stark verbacken |
| | | 5 Gew.-% Maismehl | >5x klopfen | stark verbacken |
| | | 2 Gew.-% D17 | 1x klopfen | leicht verbacken |
| | | 2 Gew.-% D17 | 1x klopfen | nicht verbacken, staubend |
| | | 1 Gew.-% Aerosil200 + 2% R972 | 1x klopfen | nicht verbacken |
| | | 2 Gew.-% Aerosil200 + 2% R972 | 1x klopfen | leicht verbacken, staubend |

| | | | | |
|---|---|---|---|---|
| ¹, ², ³, ⁴, ⁵ verschiedene Kieselsäuren der Fa. Degussa | | | | |
| ⁶ Zeolith der Fa. Degussa | | | | |

### D. Ansäuerung von Nahrungsmitteln

### Beispiel 15

Ein "Ferkelprestarterfutter" wurde mit je 10 kg/to bzw. 20 kg/t eines getränkten Salzes (NaFormiat/15 Gew.-% Ameisensäure) behandelt. Der pH-Wert des Futters sank von 6,4 auf 5,5 bzw. 5,1.

## Patentansprüche

1. Getränkte Salze enthaltend mindestens ein Salz einer oder mehrerer organischer Carbonsäuren, das mit 0,5 bis 30 Gew.-% mindestens einer flüssigen Carbonsäure bezogen auf das Carbonsäuresalz getränkt wurde.

2. Getränkte Salze nach Anspruch 1, enthaltend mindestens ein Salz einer C₁-C₈-Mono- oder Di-Carbonsäuren, die mit mindestens einer C₁-C₈-Mono- oder Di-Carbonsäuren getränkt wurde.

3. Getränkte Salze nach Anspruch 1 oder 2, enthaltend mindestens ein Salz einer Carbonsäure ausgewählt aus der Gruppe Ameisensäure, Essigsäure oder Propionsäure, das mit mindestens einer Carbonsäure ausgewählt aus der Gruppe Ameisensäure, Essigsäure oder Propionsäure getränkt wurde.

4. Getränkte Salze nach den Ansprüchen 1 bis 3, wobei die Carbonsäure in den Carbonsäuresalzen und die zur Tränkung der Salze verwendete Carbonsäure identisch ist.

5. Getränkte Salze nach den Ansprüchen 1 bis 4, **dadurch gekennzeichnet, daß** die getränkten Salze mindestens ein Salz einer oder mehrerer organischer Carbonsäuren ausgewählt aus der Gruppe der Ammonium-, Kalium-, Natrium-, Lithium-, Magnesium- oder Calciumsalze enthalten.

6. Konservierungsstoffe enthaltend ein getränktes Salz gemäß Anspruch 1.

7. Konservierungsstoffe nach Anspruch 6, enthaltend zusätzlich einen Trägerstoff und/oder Formulierungshilfsstoffe.

8. Konservierungsstoffen nach Anspruch 6 oder 7, **dadurch gekennzeichnet, daß** sie mit einem bei 20 °C wasserlöslichen oder wasserquellbaren Abdeckmittel überzogen sind.

9. Konservierungsstoffe nach den Ansprüchen 6 bis 8, **dadurch gekennzeichnet, daß** als Abdeckmittel wasserlösliche Polymere, organische Säuren, deren Salze oder niedrig schmelzende anorganische Salze verwendet werden.

10. Konservierungsstoffe nach den Ansprüchen 6 bis 9, **dadurch gekennzeichnet, daß** als Abdeckmittel Polyethylenglykole, Polyvinylpyrrolidone oder organische Säuren und deren Salze von C₃ bis C₁₄, vorzugsweise C₃ bis C₆, insbesondere Zitronensäure, Fumarsäure, Bernsteinsäure, Adipinsäure, Benzoesäure, Sorbinsäure und deren Salze oder Aminosäuren und deren Salze, verwendet werden.

11. Konservierungsstoffe nach den Ansprüchen 6 bis 10, **dadurch gekennzeichnet, daß** zusätzlich oder anstelle des Abdeckmittel ein Puderungsmittel auf der Oberfläche aufgebracht wird.

12. Verfahren zur Herstellung von getränkten Salzen gemäß Anspruch 1, **dadurch gekennzeichnet, daß** man mindestens ein Salz einer Carbonsäure oder eines Carbonsäuregemisches mit mindestens einer flüssigen Carbonsäure bis zu einer Konzentration von 30 Gew.-% bezogen auf das Carbonsäuresalz tränkt.

13. Verfahren nach Anspruch 12, **dadurch gekennzeichnet, daß** mindestens eine Carbonsäure in einem Mischer vorgelegt wird und mindestens ein Salz einer Carbonsäure oder eines Carbonsäuregemisches zu dosiert wird.

14. Verfahren zur Herstellung von Konservierungsstoffen gemäß Anspruch 6, **dadurch gekennzeichnet, daß** man getränkte Salze gemäß Anspruch 1 mit einem oder mehreren Trägerstoffen und/ oder Formulierungshilfsstoffen mischt und unter oder ohne Zugabe mindestens eines Bindemitttels agglomeriert.

15. Verfahren nach Anspruch 14, **dadurch gekennzeichnet, daß** man die Konservierungsstoffe mit einem bei 20°C wasserlöslichen oder wasserquellbaren Abdeckmittel überzieht und/oder gewünschtenfalls die Rieselfähigkeit der Konservierungsstoffe durch Abpudern mit einem feindispersen Pudermittel sichergestellt.

16. Verwendung der getränkten Salze gemäß Anspruch 1 oder der Konservierungsstoffe gemäß Anspruch 6 zur Säurebehandlung, zur Konservierung von Lebens- und Futtermitteln, zur Anwendung in Silagen oder zur Lederbehandlung.

## Claims

1. Impregnated salts comprising at least one salt of one or more carboxylic acids, which salt has been impregnated with from 0.5 to 30% by weight, based on the carboxylic acid salt, of at least one liquid carboxylic acid.

2. Impregnated salts as claimed in claim 1, comprising at least one salt of a C₁-C₈-mono- or dicarboxylic acid, which salt has been impregnated with at least one C₁-C₈-mono- or dicarboxylic acid.

3. Impregnated salts as claimed in claim 1 or 2, comprising at least one salt of a carboxylic acid selected from the group of formic acid, acetic acid or propionic acid, which salt has been impregnated with at least one carboxylic acid selected from the group of formic acid, acetic acid or propionic acid.

4. Impregnated salts as claimed in any of claims 1 to 3, where the carboxylic acids in the carboxylic acid salts and the carboxylic acid used for impregnating the salts are identical.

5. Impregnated salts as claimed in any of claims 1 to 4, wherein the impregnated salts comprise at least one salt of one or more carboxylic acids selected from the group of ammonium, potassium, sodium, lithium, magnesium or calcium salts.

6. A preservative comprising an impregnated salt as claimed in claim 1.

7. A preservative as claimed in claim 6, additionally comprising a carrier and/or formulation auxiliaries.

8. A preservative as claimed in claim 6 or 7, which is coated with a protective agent which is soluble or swellable in water at 20°C.

9. A preservative as claimed in any of claims 6 to 8, wherein water-soluble polymers, organic acids, their salts or low-melting inorganic salts are used as protective agents.

10. A preservative as claimed in any of claims 6 to 9, wherein polyethylene glycols, polyvinylpyrrolidones or C₃-C₁₄, preferably C₃-C₆, organic acids and their salts, in particular citric acid, fumaric acid, succinic acid, adipic acid, benzoic acid, sorbic acid and their salts, or amino acids and their salts, are used as protective agents.

11. A preservative as claimed in any of claims 6 to 10, wherein a dusting powder is applied to the surface in addition to or in place of the protective agent.

12. A process for producing impregnated salts as claimed in claim 1, which comprises impregnating at least one salt of a carboxylic acid or of a mixture of carboxylic acids with at least one liquid carboxylic acid until the concentration is 30% by weight based on the carboxylic acid salt.

13. A process as claimed in claim 12, wherein at least one carboxylic acid is introduced into a mixer, and at least one salt of a carboxylic acid or of a mixture of carboxylic acids is metered in.

14. A process for producing a preservative as claimed in claim 6, which comprises mixing impregnated salts as claimed in claim 1 with one or more carriers and/or formulation auxiliaries, and agglomerating with or without the addition of at least one binder.

15. A process as claimed in claim 14, wherein the preservative is coated with a protective agent which is soluble or swellable in water at 20°C and/or if required the flow characteristics of the preservative are ensured by dusting with a finely dispersed dusting powder.

16. The use of the impregnated salts as claimed in claim 1 or of the preservative as claimed in claim 6 for acid treatment, for preserving human and animal food, or for use in silage or for leather treatment.

## Revendications

1. Sels imprégnés contenant au moins un sel d'un ou plusieurs acides organiques carboxyliques, qui a été imprégné par 0,5 à 30 % de son poids d'au moins un acide carboxylique liquide.

2. Sels imprégnés selon la revendication 1, contenant au moins un sel d'un acide mono- ou di-carboxylique en C1-C8, qui a été imprégné par au moins un acide mono- ou di-carboxylique en C1-C8.

3. Sels imprégnés selon la revendication 1 ou 2, contenant au moins un sel d'un acide carboxylique choisi dans le groupe formé par l'acide formique, l'acide acétique et l'acide propionique, qui a été imprégné par au moins un acide carboxylique choisi dans le groupe de l'acide formique, de l'acide acétique ou de l'acide propionique.

4. Sels imprégnés selon les revendications 1 à 3, pour lesquels l'acide carboxylique des sels d'acides carboxyliques et l'acide carboxylique utilisé pour l'imprégnation des sels sont identiques.

5. Sels imprégnés selon les revendications 1 à 4, **caractérisés par le fait qu'**ils contiennent au moins un sel d'un ou plusieurs acides organiques carboxyliques choisi dans le groupe des sels d'ammonium, de potassium, de sodium, de lithium, de magnésium ou de calcium.

6. Conservateurs contenant un sel imprégné selon la revendication 1.

7. Conservateurs selon la revendication 6, contenant en outre un véhicule et/ou des produits auxiliaires de formulation.

8. Conservateurs selon la revendication 6 ou 7, **caractérisés par le fait qu'**ils sont revêtus d'un produit de couverture soluble dans l'eau ou gonflable à l'eau à 20°C.

9. Conservateurs selon les revêtements 6 à 8, **caractérisés par le fait que** l'on utilise en tant que produits de couverture des polymères solubles dans l'eau, des acides organiques, leurs sels ou des sels minéraux à bas point de fusion.

10. Conservateurs selon les revendications 6 à 9, **caractérisés par le fait que** l'on utilise en tant que produits de couverture des polyéthylèneglycols, des polyvinylpyrrolidones ou des acides organiques en C3-C14, de préférence en C3-C6, et leurs sels, en particulier l'acide citrique, l'acide fumarique, l'acide succinique, l'acide adipique, l'acide benzoïque, l'acide sorbique et leurs sels ou des aminoacides et leurs sels.

11. Conservateurs selon les revendications 6 à 10, **caractérisés par le fait qu'**ils portent, appliqué en surface, en plus du produit de couverture ou à la place du produit de couverture, un produit de poudrage.

12. Procédé pour la préparation de sels imprégnés selon la revendication 1, **caractérisé par le fait que** l'on imprègne au moins un sel d'un acide carboxylique ou d'un mélange d'acides carboxyliques par au moins un acide carboxylique liquide jusqu'à une concentration de 30 % du poids du sel d'acide carboxylique.

13. Procédé selon la revendication 12, **caractérisé par le fait que** l'on introduit au moins un acide carboxylique dans un mélangeur et on ajoute au moins un sel d'un acide carboxylique ou d'un mélange d'acides carboxyliques.

14. Procédé pour la préparation de conservateurs selon la revendication 6, **caractérisé par le fait que** l'on mélange des sels imprégnés selon la revendication 1 avec un ou plusieurs véhicules et/ou produits auxiliaires de formulation et on agglomère en ajoutant ou non au moins un liant.

15. Procédé selon la revendication 14, **caractérisé par le fait que** l'on revêt les conservateurs par un produit de couverture soluble dans l'eau ou gonflable à l'eau à 20°C et/ou, si on le désire, on assure des bonnes qualités d'écoulement des conservateurs par poudrage à l'aide d'un produit de poudrage finement dispersé.

16. Utilisation des sels imprégnés selon la revendication 1 ou des conservateurs selon la revendication 6 pour un traitement d'acidification, pour la conservation de produits alimentaires humains et animaux, pour l'utilisation dans des ensilages ou pour le traitement des cuirs.
